# EUROPEAN PATENT SPECIFICATION

(11) **EP 0 806 668 B1**
(45) Date of publication and mention of the grant of the patent: **31.07.2002**
(21) Application number: 97107424.0
(22) Date of filing: 06.05.1997
(51) Int. Cl.: G01N 33/574

(54) **Method and kit for determining malignant potential in tissue cells**
Verfahren und Vorrichtung zum Nachweis eines malignen Potentials in Gewebezellen
Procédé et dispositif pour détecter un potential maligne dans des cellules de tissu

(30) Priority: 10.05.1996 US 644743
(43) Date of publication of application: 12.11.1997
(73) Proprietor: Becton, Dickinson and Company, Franklin Lakes, New Jersey 07417-1880 (US)
(72) Inventor: Ramaekers, Frans C.S., 6213 CB Maastricht (NL)
(74) Representative: Gerbino, Angelo

(56) References cited:
- WO-A-95/22114
- DER PATHOLOGE, vol. 15, February 1994, BERLIN, pages 337-344, XP002041079 PÖSL ET AL.: "Osteosarkom - Apoptose und proliferation"
- JOURNAL OF PATHOLOGY, vol. 168, 1992, CHICHESTER, pages 357-363, XP002041080 CATTORETTI ET AL.: "Monoclonal antibodies against recombinant parts of the Ki-67 antigen (MIB 1 and MIB 3) detect proliferating cells in microwave-processed formalin-fixed paraffin sections"
- HUMAN PATHOLOGY, vol. 27, no. 2, February 1996, PHILADEPHIA, PA, pages 102-110, XP002041081 LU ET AL.: "bcl-2: role in epithelial differentiation and oncogenesis"
- INTERNATIONAL JOURNAL OF GYNECOLOGICAL PATHOLOGY, vol. 15, no. 1, January 1996, NEW YORK, NY, pages 10-16, XP002041082 HOEVEN ET AL.: "Immunohistochemical staining for proliferating cell nuclear antigen, BCL2, and Ki-67 in vulvar tissues"
- INTERNATIONAL JOURNAL OF GYNECOLOGICAL PATHOLOGY, vol. 13, no. 4, 1994, NEW YORK, NY, pages 337-341, XP002041083 RAJU: "Expression of the proliferating cell nuclear antigen in cervical neoplasia"
- JOURNAL OF PATHOLOGY, vol. 179, no. 1, May 1997, CHICHESTER, pages 26-30, XP002041281 HARMSEL ET AL.: "BCL-2 immunoreactivity increases with severity of CIN: A study of normal cervical epithelia, CIN, and cervical carcinoma"

## Description

### FIELD OF THE INVENTION

This invention is in the field of medical diagnostics and prognostics, specifically, relating to a method and kit for identifying premalignant changes in the cells of epithelial tissue and predicting the progressive potential of the cells to become malignant

### BACKGROUND OF THE INVENTION

In a mature animal, a balance between cell proliferation (cell renewal) and cell death is maintained in most organs and tissues. Generally, mature cells have a given lifespan, depending upon their type, and as the mature cells die, new cells are generated by proliferation and differentiation of stem cells. Under normal conditions, cell proliferation is regulated such that the numbers of any particular cell type remain constant, thus cell proliferation and cell death are in balance. If cells which are not responsive to normal growth control mechanisms arise, clones of cells arise that can expand in size and numbers beyond that which is considered normal and may thus produce a tumor, or neoplasm. A tumor that continues to grow and become increasingly invasive in the surrounding tissue is malignant, or cancerous.

Because of the similar embryonic origin, most epithelial carcinomas exhibit similar cell characteristics. Moreover, epithelial carcinomas demonstrate a full range of preneoplastic stages.

A substantial body of work has been compiled which attempts to determine the progressive nature of premalignant cells to become malignant. However, it often is not possible to identify cell lesions which will follow a progressive course or to distinguish them from those which are regressive in nature based only on pure morphological criteria.

The accelerated proliferation of cells is a known indicator of neoplasia. Methods for determining the progressive nature of premalignant lesions include, next to a morphological examination of nuclear and cytoplasmic organization, the immunohistochemical detection of cellular markers. These markers often represent cytoplasmic or nuclear constituents which indicate the proliferative potential of the tumor cells under examination. For example, an increased number of cells expressing Ki-67-Antigen or PCNA, or showing an increased incorporation of [³H]-thymidine or BrdU, indicates a higher malignant potential or advanced stage of (pre)neoplasia. Also, the DNA content of a certain cell fraction, as determined by DNA-flow cytometry, may be used as a prognosticator.

Recently, it has become evident that not only the rate of cell renewal is important for tumor progression, but that also a decreased rate of cell death (apoptosis) may be an important factor in this respect. Cells that do not die (have become immortal) can accumulate genetic damage and thereby progress to more advanced stages of (pre)malignancy.

Methods for determining the expression of antigens such as Ki-67 Antigen and the proliferating cell nuclear antigen (PCNA) have been successfully used to identify the extent of cell proliferation. Proliferating cells staining positive for the antigens are significantly higher in premalignant and malignant lesions than in nonneoplastic lesions. Raju, G.C. Expression of the Proliferating Cell Nuclear Antigen in Cervical Neoplasia. *Int. J. Gynecol. Path*. (1994) 13:337-41. Evaluation of the expression of such indicators is used in conjunction with histological diagnosis to determine the pathogenesis of neoplasias.

The expression of cell proliferation markers and its relationship to prognosis in primary cervical cancers suggests that the percentage of proliferating cells measured immunohistochemically might be applicable to examine the biological growth potential in certain cancers and possibly indicate the clinical prognosis of the tumor. Other studies, however, have found such markers to be of limited prognotic value. Brown et al., *Carcinoma of the Cervix Uteri: An Assessment of Tumor Proliferation Using the Monoclonal Antibody Ki-67,"* Br J. Cancer 57:178-181, (1998), and Raju.

Blocked apoptosis (cell death) plays a role in tumor progression by promoting the survival of therapy-resistant variants. Smets, L. Apoptosis and cancer: Hype or Hypothesis? *LCC Newsletter* (1995), pages 10-12. Membrane protein BCL-2 is consistently expressed in normal epithelial cells and its expression is associated with inhibition of apoptosis. Siziopikou, K.P., Prioleau, J.E., Harris, J.R & Schnitt, S.J. BCL-2 Expression in the Spectrum of Preinvasive Breast Lesions. *Cancer* (1996) 77(3):499-506.

BCL-2 is involved in the regulation of cell death and survival without affecting cell proliferation. The protein has no direct effect on cell proliferation because overexpression of BCL-2 does not inevitably result in an increase in cell proliferation or alter the pattern of the cell cycle. Lu,Q.-L., Abel, P., Foster, C., & Lalani, E.-L. bcl-2: Role in Epithelial Differentiation and Oncogenesis. *Human Path*., (1996) 27(2):102-10.

The cell proliferation and apoptosis characteristics of cervical neoplasms are exemplary of most epithelial carcinomas, which account for approximately 80 - 90% of tumors occurring *in vivo*. Cervical carcinoma originates from cervical intraepithelial neoplasia (CIN) and is usually located at the squamocolumnar junction of the uterine cervix. Frequently, CINs increase in severity and become malignant. Vooijs, G.P., Benign proliferative reactions, intraepithelial neoplasia and invasive cancer of the uterine cervix. *In: Comprehensive Cytopathology*. Edited by Bibbo, M., pp. 153-230. Philadelphia/London/Toronto: W.B. Saunders Co., 1991.

CINs are classified as low- (CIN I), moderate- (CIN II), or high-grade (CIN III), depending upon their malignant potential. Low-grade CIN (CIN I) is suggested to have a low malignant potential, i.e. the risk of progression to cervical carcinoma is low. Most low-grade CIN lesions are thought to regress spontaneously. High-grade CIN (CIN III) has greatly increased malignant potential. It has been proposed that nearly 100% of high-grade CINs progress to carcinoma. Ostor, A.G. Natural history of cervical intraepithelial neoplasia: a critical review. *Int. J. Gynecol. Pathol*. (1993) 12:186-92.

The reactivity of premalignant lesions and carcinomas with panels of antibodies which have been shown to indicate specific features of malignant progression, such as markers of the cell cycle, proto-oncogenes or tumor suppressor genes are well documented. For example, the monoclonal antibody MIB 1 detects the Ki-67 Antigen (Ki-67-Ag) present in cells in all active phases of the cell cycle (G₁, S, G₂, M), but is absent in G₀. Cattoretti, G., Becker, M., Key, G., Duchrow, M., Schluter, C., Galle, J. and Gerdes, J. Monoclonal antibodies against recombinant parts of the Ki-67 antigen (MIB 1 and MIB 3) detect proliferating cells in microwave-processed formalin-fixed paraffin sections. *J. Pathol.* (1992) 168:357-63; Gerdes, J., Lemke, H., Baisch, H., Wacker, H., Schwab, U., Stein, H. Cell cycle analysis of a cell proliferation associated human nuclear antigen-defined by the monoclonal antibody Ki-67.*J. Immunol*. (1984) 133:1710-15.

The MIB 1 antibody has been shown to be extremely useful in the evaluation of proliferative activity as a determinant of Ki-67 expression in normal tissues and malignant tumors. Hendricks, J., Wilkinson, E., Kubilis, P., Drow, P., Blaydes, S., Munakata, S. Ki-67 expression in vulvar carcinoma. *Int. J. Gynecol. Pathol*. (1994) 13:205-10; Brown, D., Cole, D., Gatter, K., Mason, D. Carcinoma of the cervix uteri: An assessment of tumour proliferation using the monoclonal antibody Ki-67. *Br. J. Cancer* (1988) 57:178-81. Studies of proliferative activity in gynecological tumors suggest that the pattern of antigen expression is associated with aggressive behavior. Pattern determination of antigen staining alone, however, offers a somewhat arbitrary indicator of the progressive nature of a tumor mass that may be complicated by the presence and/or extent of localized expression patterns.

Proliferation markers have been examined to determine the proliferative activity of the cells in cervical lesions (see Raju, Gangaraju C., Expression of the Proliferating Cell Nuclear Antigen in Cervical Neoplasia; International Journal of Gynaecological Pathology, 1994, 13:337-341), without however combining the co-assessment of a proliferation marker with another marker. Bcl-2 has been disclosed as a marker for epithelial malignancies (see Lu, Qi Long, Abel, Paul, Foster, Christopher, Lalani, El-Nasir, bcl-2: Role in Epithelial Differentiation and Oncogenesis; Human Pathology, 1996, 27(2):102-110). There are some teachings suggesting the use of multiplemarkers in the characterisation of excised tumours (see Posl, M., Amling, M., Werner, M., Basler, M. Salzer-Kuntschik, M., Winkler, KJ., Delling, G., Osteosarkom Apoptose und Proliferation, Pathologe, 1994, 15:337-344), without nevertheless suggesting the combination of both a proliferation marker together with a marker of programmed cell death for the detection of pre-malignant changes in epithelial tissues.

Although such immunocytochemical techniques are valuable to the study of various cell characteristics of cancers, individual antigens and/or antigen markers have so far not been successfully used to predict the progressiveness of a cell lesion or tumor when the cells are premalignant. There remains a need for a method for identifying early premalignant changes in cells which accurately determine the progressive potential of these preneoplastic stages.

### SUMMARY OF THE INVENTION

The present invention relates to a method as indicated in claim 1 for measuring simultaneously cell proliferation and cell apoptosis inhibition in epithelial tissues to identify premalignant cell changes which may be used to predict the progressive potential of the cells to transform to cancer. In a preferred embodiment of the method, the level of cell proliferation is measured by determining the expression of a cell proliferation antigen, such as the Ki-67-Ag. Ki-67-Ag expression may be determined immunocytochemically with the MIB 1 antibody. The level of cell death inhibition may be measured by determining the expression of the BCL-2 cell membrane protein.

The present invention determines the combination of both markers in the same cell population. As described below in more detail; normal cervical epithelium (ectocervix) contains a fraction of proliferative cells (the parabasal cell layer) and a fraction of cells that are protected against apoptosis (the basal cell layer). Thus, a normal epithelium topographically separates both cell fractions, and the percentage of cells combining a high proliferative potential with cell death inhibition is very low. With increasing severity of preneoplasia, the number of cells combining both parameters increases dramatically, increasing up to 80% or more.

Cell proliferation and cell apoptosis inhibition markers may be measured using tissue slides which are immunostained to detect simultaneously an indicator of cell proliferation and an indicator of cell death inhibition. In alternate embodiments of the present invention, smears of the cells are prepared and immunostained to detect simultaneously an indicator of cell proliferation and an indicator of cell death inhibition as a measure of cell proliferation and a measure of cell death inhibition. In yet other embodiments, the level of cell proliferation and the level of cell death inhibition is measured simultaneously by flow cytometry or morphometric imaging.

### BRIEF DESCRIPTION OF THE DRAWINGS

Figure 1 shows an immunohistochemical staining of normal ectocervix tissue with the Ki-67-Ag antibody MIB-1.

Figure 2 shows an immunohistochemical staining of normal ectocervix tissue with the BCL-2 antibody 124 mAb.

Figure 3 shows an immunohistochemical staining of cervical intraepithelial neoplasia (CIN III) with MIB-1.

Figure 4 shows an immunohistochemical staining of cervical intraepithelial neoplasia (CIN III) with the BCL-2 antibody.

Figure 5a illustrates the relationship between proliferative cells and cells protected from cell death in normal epithelium.

Figure 5b illustrates the increase in cells combining high proliferative potential with cell death inhibition with increasing severity of preneoplasia.

Figure 6 is a schematic representation of the average percentages of Ki-67-Ag and BCL-2 immunostaining cells in normal, preneoplastic and neoplastic cervical epithelia.

### DETAILED DESCRIPTION OF THE INVENTION

The present invention relates to a method and kit for measuring simultaneously cell proliferation and cell apoptosis inhibition in epithelial tissues to identify premalignant cell changes which may be used to predict the progressive potential of the cells to transform to cancer. The present invention determines the combination of both markers in the same cell population, thereby providing a comparative analysis for determining the malignant potential of premalignant cells.

In a preferred embodiment of the present invention; the cytokinetic parameters for the progression of preneoplasia are determined by measuring the expression of a cell proliferation marker in normal, metaplastic, and preneoplastic epithelia. The capacity of dysplastic epithelial cells to withdraw from programmed cell death (apoptosis) is also measured in the same specimen. The results of cell proliferation measures and inhibition of cell death (apoptosis) measures are then correlated to the morphologically determined degree of (pre)neoplasia. Determinations of cell proliferation and inhibition of apoptosis may be measured in any epithelial tissue, lesions or carcinomas according to the method described herein.

While the following discussion of the present invention pertains to the determination of cell proliferation and inhibition of apoptosis in cervical tissue, CIN lesions, and cervical carcinomas, the present invention can be used to determine cell proliferation and inhibition of apoptosis in any tissue of epithelial cells, lesions, and carcinomas.

The protocol and techniques described below were used to simultaneously measure cell proliferation and cell apoptosis inhibition in the cervical specimens. The protocol and techniques described can successfully be used to practice the invention using other tissue specimens. Therefore, the discussion as it pertains to cervical specimens is exemplary and not limiting.

The balance between the proliferative cell fraction and the cell fraction protected from apoptosis of normal cervical epithelia, CIN lesions and cervical carcinomas was determined by measuring Ki-67-Ag expression and BCL-2 expression. Cell proliferation was measured using the MIB 1 antibody to detect the Ki-67-Ag. The expression of the membrane protein BCL-2, was measured as determinative of protection against apoptosis in the cells of the epithelia.

BCL-2 protects cells from apoptosis and is, therefore, expressed in stem cell compartments and other cells in which protection from cell death is important for tissue homeostasis. Hokenberry, D., Zutter, M., Hickey, W., Nahm, M., Korsmeyer, S. BCL-2 protein is topographically restricted in tissues characterized by apoptotic cell death. *Proc. Natl. Acad. Sci. U*.*S*.*A*. (1991) 88:6961-65; Hockenbery, D., Nuffez, G., Milliman, C., Schreiber, R., Korsmeyer, S. BCL-2 is an inner mitochondrial membrane protein that blocks programmed cell death. *Nature* (1990) 348:334-36. In premalignant lesions, accumulation of BCL-2 protein can lead to a prolonged life-span of cells, which may result in an accumulation of genetic aberrations in cells which are highly protected against cell death. Thus, the cells having expanded lifespans as well as a high proliferative capacity, continue to give rise to clones of cells that may produce a malignant tumor.

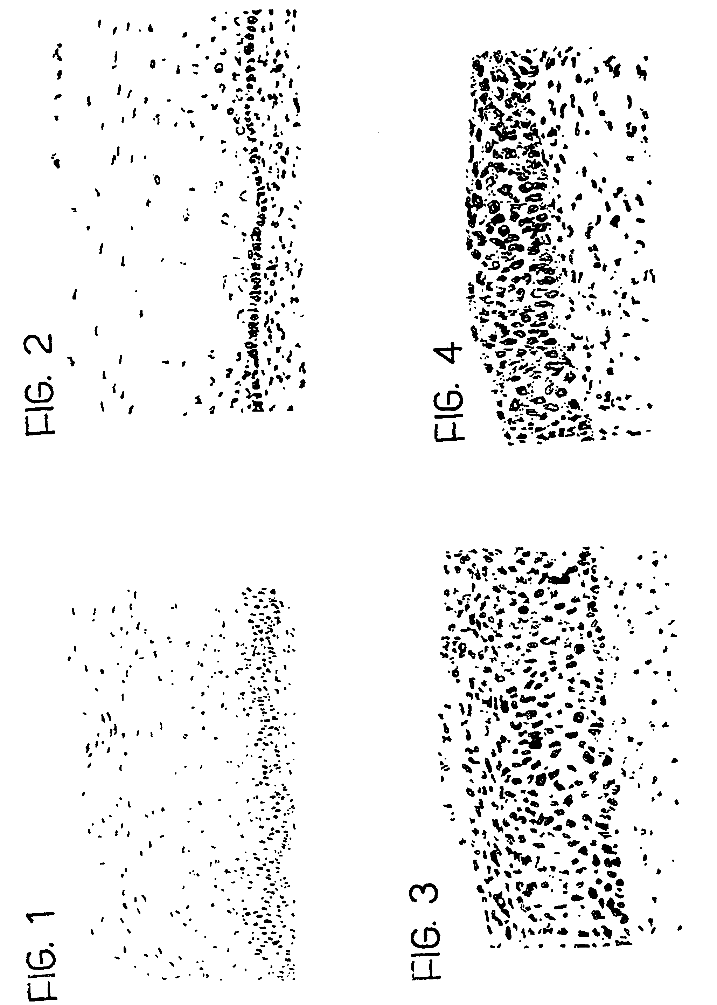

Ki-67-Ag and BCL-2 expression were determined in formalin fixed and paraffin embedded cervical tissue specimens. The tissue specimens included excision biopsies from the normal cervix taken from hysterectomy specimens removed for benign conditions. These biopsies served as controls (n=16). The parabasal cell layer of proliferative cells in normal cervical epithelium (ectocervix) is shown generally in Figure 1. The basal cell layer including a fraction of cells protected against cell death (apoptosis) in normal cervical epithelium (ectocervix) is shown generally in Figure 2.

For cervical intraepithelial neoplasia (CIN) lesions, diathermy loop excision specimens were used. These specimens were removed from women with cytologically verified CIN. The specimens consisted of 16 cases of CIN I, 12 cases of CIN II, and 13 cases of CIN III. Tissue samples from 8 cervical squamous cell carcinomas and 5 adenocarcinomas were taken from radical hysterectomy specimens.

In some cases, more than one type of epithelium was investigated in a tissue specimen. By doing so, 29 cases with normal ectocervical non-keratinizing epithelium; 37 samples with normal endocervical epithelium; 20 samples with normal endocervical reserve cells; 8 cases of immature squamous metaplasia; and 13 cases of mature squamous metaplasia were observed. The histologic diagnosis was performed on hematoxylin-eosin (H&E) stained slides using standard staining procedures known to those skilled in the art.

The specimens were prepared as follows for determining Ki-67-Ag expression. Immunocytochemical staining procedures were performed using the MIB 1 antibody (available from Immunotech, Marseille, France) as a marker of Ki-67-Ag expression. 2-µm thick sections were cut from representative paraffin blocks, mounted on organosilane coated slides and dried overnight. After deparaffination and blocking of endogenous peroxidase activity, the sections were incubated in citrate buffer (0.01 M, pH=6.0) for two consecutive periods of 5 minutes in a microwave oven (Panasonic) at 750 W. Between the two periods, the volume of citrate buffer was checked and adjusted when needed. The sections were then incubated with the undiluted antibody MIB 1 for 1 hour at room temperature. After subsequent washing steps with phosphate buffered saline (PBS), the peroxidase labeled Strept-Avidin detection system(BioGenex Laboratories, San Ramon, California, U.S.A.) was applied to the sections. The peroxidase activity was detected with diamine-benzidine HCl (DAB). The sections were counterstained, dehydrated and mounted with coverfilm.

The immunohistochemical staining procedure for BCL-2 was essentially the same as described for MIB 1. The monoclonal antibody 124 mAb (available from DAKO A/S, Glostrup, Denmark), was diluted 1:50 and the signal visualized by the indirect avidin-biotin procedure (High Performance Multi Link Kit, BioGenex, San Ramon, California, U.S.A.) using DAB as a chromogen.

Positively staining normal lymphocytes infiltrating the stroma of the cervical lesions represented an internal positive control for BCL-2 immunostaining. Furthermore, positive controls for MIB 1 and BCL-2 consisted of lymph node tissue reactive follicular hyperplasia. For BCL-2. the germinal center cells of the lymphoid follicle were negative and most of the follicle mantle zone cells were positive, as described elsewhere. Gerdes, J., et al., *Cell Cycle Analysis of a Cell* *Proliferation-Associated Human Nuclear Antigen Defined by the Monoclonal Antibody Ki-67. J. Immunol*. (1984) 133:1710-15.

All H&E stained and immunostained slides were evaluated for MIB 1 and BCL-2 levels using a light microscope. MIB 1 staining was in general strong and was evaluated by determining the number of positive nuclei in each type of epithelium in relation to the total number of nuclei. In this way the percentage of positively staining nuclei was determined in each epithelial compartment. In some cases minor cytoplasmic staining was observed, which was recorded separately. BCL-2 showed cytoplasmic staining which was graded strong, moderate, weak and very weak. Furthermore, the percentage of cells staining positive for BCL-2 in each epithelial compartment was scored.

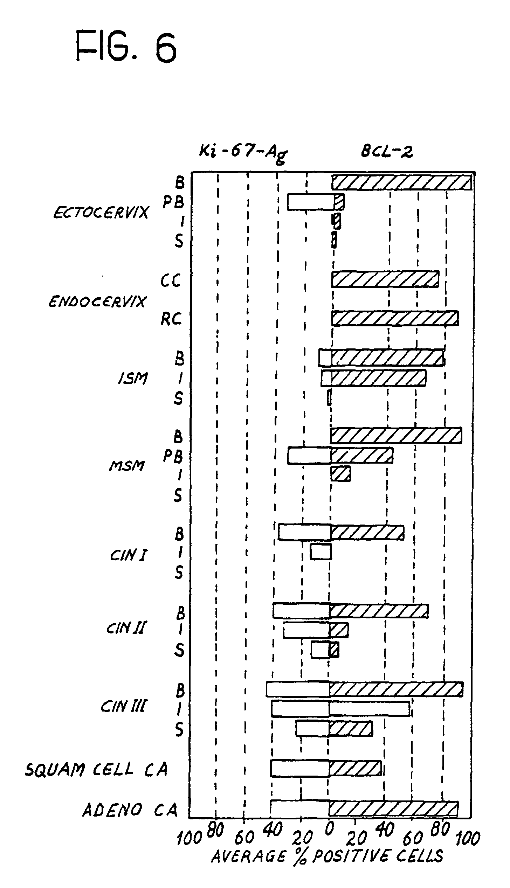

The results reported below are summarized in Figure 6. The abbreviations along the left margin of the table are defined as: basal cell compartment (B); parabasal cell compartment (PB); intermediate cell compartment (I); superficial cell compartment (S); columnar cells (CC); reserve cells (RC); immature squamous metaplastic epithelium (ISM); mature squamous metaplastic epithelium (MSM).

Referring to Figure 1, Ki-67-Ag expression, as detected by MIB 1, was prominently present in nuclei of cells in the parabasal cell layer of normal, ectocervical non-keratinizing squamous epithelium. An average of 34% of nuclei in this cell compartment stained positively, the range varying between 10 and 60%. Only sporadic nuclei (less than 1%) in the basal cell compartment were positive for Ki-67-Ag. The intermediate cell compartment and superficial cell compartment were completely negative for Ki-67-Ag. BCL-2 was identified in >95% of the cells in the basal cell compartment of all specimens (see Figure 2). In the overlying epithelial layers, a minor fraction of the cells stained weakly.

Ki-67-Ag was observed in less than 1% of endocervical columnar cells in 12 of 37 tissue fragments. In the cases of positive staining, the cell cytoplasm also displayed some immunoreactivity. BCL-2 immunoreactivity was observed in approximately 75% of the cells in these specimens and was usually weak. The other specimens were negative for BCL-2.

Ki-67-Ag was detected in 2 of the 20 specimens with reserve cells. One specimen showed approximately 20% of the nuclei positive for Ki-67-Ag and the other showed only 1% of the nuclei positive for Ki-67-Ag. BCL-2 was detected in nearly all reserve cells in all tissue specimens. Staining was usually moderate.

Of the eight tissue specimens in which immature squamous metaplastic epithelium was diagnosed, four specimens were from the control group and four specimens were associated with CIN lesions. Ki-67-Ag was expressed in all 8 cases, however, in the control specimens approximately 5% of the basal cell nuclei were positive for Ki-67-Ag. In the immature metaplastic epithelium associated with CIN, approximately 15% of nuclei in the basal cell layer were stained. In 6 of the eight specimens, up to approximately 7% of nuclei in the intermediate cell compartment stained positive. In one of the control specimens, approximately 10% of the nuclei in the superficial cell compartment stained positive for Ki-67-Ag. BCL-2 staining in immature squamous metaplastic epithelium was variable, with all cases showing weak to moderate cytoplasmic staining in the basal or intermediate cell compartments.

The results of staining with antibodies for MIB 1 and BCL-2 in mature squamous metaplasia specimens were identical to that observed in normal ectocervical non-keratinizing squamous epithelium.

CIN specimens are shown in Figures 3 and 4. The Ki-67-Ag was expressed in all 16 tissue fragments with CIN I. The percentage of positively staining nuclei varied considerably between the individual specimens. In the basal cell compartment, nuclear staining varied from approximately 10 to 70% of the cells with approximately 40% of the nuclei staining in the majority of cases. In 12 cases, staining was noted in approximately 5 to 50% of nuclei of the intermediate cell compartment. Staining of the superficial cells was noted in 3 of 16 cases, all showing less than approximately 5% of the nuclei positive for Ki-67-Ag.

In CIN I, BCL-2 was immunodetected in an average of 50% of the cells in the basal cell compartment only, in all tissue specimens examined.

The 12 CIN II tissue specimens showed nuclear positivity for Ki-67-Ag in the basal cell compartment, with an average of approximately 40% of the nuclei with MIB 1 staining. The specimens exhibited a variation of between approximately 20 to 70% of the nuclei staining with MIB 1. In all cases, the intermediate cell compartment was stained, with the number of positive nuclei varying between 20 and 70% (average 33%). In the superficial compartment, between I and 70% of the nuclei stained for the Ki-67-Ag, with an average of 13%. Only one specimen was negative for Ki-67-Ag expression in the superficial compartment.

When examined for BCL-2, the basal cell compartment of CIN II showed moderate expression in all tissue specimens. In 2 tissue fragments, weak and moderate staining was seen in the intermediate compartment, while one case stained throughout the full epithelial thickness.

All 13 CIN III tissue fragments displayed immunoreactivity for Ki-67-Ag. In the basal cell compartment, an average of 45% of the nuclei stained positive, demonstrating variation of between approximately 1 and 70% of the nuclei positively staining (see Figure 3). In the intermediate and superficial cell compartment, 12 of 13 specimens showed positive Ki-67-Ag expression in approximately 1 to 70% of the nuclei. The average percentage of nuclei staining positive for Ki-67-Ag in the intermediate cell compartment and the superficial cell compartment layer was approximately 40% and 24%, respectively.

In all CIN III lesions, approximately 95% of the cells in the basal cell compartment were moderately immunodetected by the BCL-2 antibody. In the intermediate cell compartment, 11 fragments showed immunoreactivity in approximately 60% of cells (see Figure 4). In 5 lesions, there was also weak BCL-2 immunoreactivity in the superficial layer in approximately 30% of the cells. Three lesions showed high levels of Ki-67-Ag and BCL-2 in all cell layers.

The carcinoma tissue specimens comprised 8 non-keratinizing squamous cell carcinomas and 5 adenocarcinomas of the cervix. The Ki-67-Ag antibody was expressed in all carcinomas, with an average of 40% of all nuclei staining positively. An average range from approximately 5 to 70% of nuclear staining positive was similar for both tumor types. It was noted that Ki-67-Ag expression increased with adenocarcinoma grade.

BCL-2 was expressed in all adenocarcinomas, showing weak to moderate staining in approximately 90% of malignant cells. The squamous cell carcinomas showed weak BCL-2 immunoreactivity in 5 of 8 cases, with an average of 38% of the cells staining positively.

The results of Ki-67-Ag expression and apoptosis inhibition as indicated by BCL-2, summarized in Figure 6, were compared to morphologically determined stages of premalignant changes, which serve as predictors of progressive potential.

The high Ki-67-Ag expression levels seen in the nuclei of the parabasal cell compartment of ectocervical epithelium reflects that this compartment harbors the highest cell growth fraction. In contrast, the basal cell compartment shows a low proliferative fraction. A striking difference was observed in immature squamous metaplasia and CIN as compared to normal ectocervical and mature squamous metaplastic epithelium with respect to expression of the Ki-67-Ag in basal and parabasal cell nuclei. The former epithelia all show pronounced proliferative activity in their basal cell compartments as concluded from their Ki-67-Ag expression, while in the latter, the proliferative compartment is limited to the parabasal cell compartment only.

An increase in Ki-67-Ag expression in the successive grades of CIN was seen. Both the number of Ki-67-Ag positive nuclei and the extent of staining in the superficial layers increased with progression of CIN. The increase in the average number of Ki-67-Ag positive nuclei indicates that the extent of the growth fraction in the various CIN lesions is in its own, a useful parameter capable of indicating the severity of CIN.

A few of the specimens of low-grade CIN showed higher proliferative fractions than the average proliferative fraction of high-grade CIN. Moreover, some cases of high-grade CIN lesions showed lower numbers of Ki-67-Ag positive nuclei than observed in most CIN I lesions. This suggests that lesions morphologically classified as CIN I with high levels of Ki-67-Ag may have a more progressive potential than CIN III with a low proliferative fraction.

It was also observed that immature squamous metaplastic lesions associated with CIN showed higher levels of Ki-67-Ag staining than immature squamous metaplastic lesions seen in controls. This appears to be an early indication that increased proliferation, as indicated by the extent of Ki-67-Ag expression in the immature squamous epithelium, precedes the morphologic changes characteristic of CIN. Thus, this epithelium will not follow the usual route of differentiation to mature metaplastic epithelium as observed in control specimens, but will develop into CIN.

The level of Ki-67-Ag expression in immature squamous metaplasia was, however, found to be considerably lower than in the low grade CIN lesions. This indicates that these two types of epithelium may be easily separated on the basis of their levels of Ki-67-Ag expression.

BCL-2 expression in the basal cells of the ectocervix reflects the stem cell function of this compartment. It appears that BCL-2 is required for protection of these stem cells against apoptotic cell death, thereby maintaining epithelial homeostasis. The presence of BCL-2 in the basal cells, as well as in the reserve cells, representing cell compartments with low proliferative capacity, is necessary for maintaining these epithelia with stem cell characteristics.

BCL-2 expression increases with increasing severity of CIN, and in this way an extending cell compartment arises that combines both Ki-67-Ag and BCL-2 expression. In cases in which both markers are intensely expressed, protection from apoptotic cell death and an increased growth fraction have a synergistic effect in the accumulation of dysplastic cells. In these lesions, the accumulation of genomic aberrations is likely to occur since cells that acquire irreversible genetic damage during DNA replication are no longer prone to apoptosis.

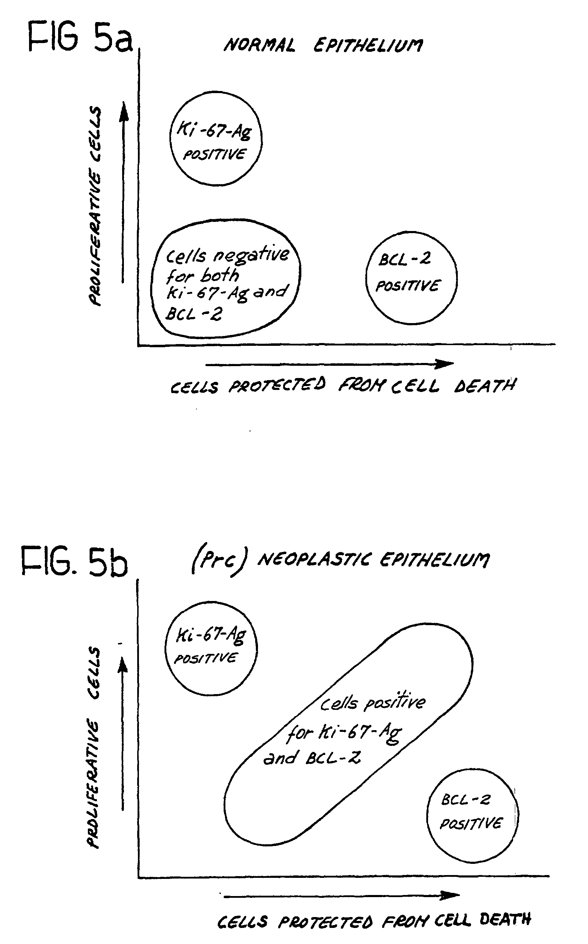

The staining patterns of the cell proliferation and apoptosis protection markers in epithelial tissues and (pre)cancerous lesions provide important information on the progressive potential of CIN and on the prognosis in patients suffering from cervical carcinomas. The relationship between proliferative cells and cells protected from cell death in normal epithelium, determined using Ki-67 antigen and BCL-2 as markers, respectively, is illustrated generally in Figure 5a. The combined expression of the above-mentioned markers within one and the same cell population indicates the presence of a potentially progressive and aggressive fraction within an epithelium. This relationship is illustrated in Figure 5b. Furthermore, the percentages of cells co-expressing proliferation and apoptosis protection markers indicates the severity of the lesion under examination.

In another embodiment of the present invention, the progressive potential of premalignant tissue cells may be determined using a prognostic immunohistochemical kit comprising an antibody capable of distinguishing epithelial cells from non-epithelial cells, an antibody which detects a cell proliferation marker, and an antibody which detects a cell apoptosis protection marker. An example of an antibody capable of distinguishing epithelial cells from non-epithelial cells is an antibody for detecting or marking keratin in the epithelial cells. Since non-epithelial cells do not contain keratin, these are gated out from a cell/tissue preparation In another embodiment, epithelial cells are distinguished from non-epithelial cells negatively, i.e., by detecting a marker not found in epithelial cells, for example, by using CD45. Other antibodies useful. to detect pan-hematopoietic cells are also useful.

Each antibody is tagged with a different label. Examples of suitable labels include enzymes, such as Horseradish Peroxidase (HRP), Fluorochromes, such as FITC, or other ligands, such as biotin, so that three parameters, i.e., epithelial cell marker, cell proliferation marker, and cell apoptosis inhibition marker, are labeled differently. The triple-labeled tissue cell specimen may be analyzed using flow cytometry methods or static tissue analysis techniques.

Numerous antibodies known to those skilled in the art may be selected for keratin labeling. Exemplary of those antibodies are RCK108, RCK102, and RCK105. Ki-67-Ag and BCL-2 may be used as cell proliferation and cell apoptosis inhibition markets, respectively. The antibodies described above for identifying these markers may be used in the kit, as well as others known to those skilled in the art.

Although the foregoing invention has been described in some detail by way of illustration and example for purposes of clarity and understanding, it will be obvious that certain changes and modifications may be practiced within the scope of the invention, as limited only by the scope of the appended claims.

## Claims

1. A method performed in vitro of assessing the potential of cervical epithelial tissue cells to progress to carcinoma, comprising:
comparing a first percentage of cells from a sample of said cervical epithelial tissue that coexpress a first marker of cellular proliferation and a second marker of programmed cell death inhibition to a percentage of cells in a cellular standard that coexpress said first and second markers,
whereby with increasing severity of preneoplasia, the number of cells combining both parameters increases thereby indicating the progressive potential of said tissue cells to progress to carcinoma.

2. The method of claim 1 wherein said marker of cellular proliferation is a cell proliferation antigen.

3. The method of claim 2 wherein said cell proliferation antigen is the Ki-67 antigen.

4. The method of claim 3 wherein the Ki-67 antigen is detected using an antibody that specifically recognizes said antigen.

5. The method of claim 4 wherein said antibody is MIB-1.

6. The method of claim 1 wherein said marker of programmed cell death inhibition is a programmed cell death inhibition antigen.

7. The method of claim 6 wherein said programmed cell death inhibition antigen is BCL-2 protein.

## Patentansprüche

1. Verfahren, das in vitro durchgeführt wird, zur Abschätzung des Potentials von zervikalen Epithelgewebezellen, sich zu einem Karzinom zu entwickeln, umfassend:
den Vergleich eines ersten Prozentsatzes an Zellen aus einer Probe des zervikalen Epithelgewebes, die einen ersten Marker einer zellulären Wucherung und einen zweiten Marker einer programmierten Zelltodhemmung coexprimieren, mit einem Prozentsatz an Zellen in einem zellulärren Standard, die den ersten und zweiten Marker coexprimieren,
wobei mit zunehmender Schwere der Präneoplasie die Anzahl an beide Parameter kombinierenden Zellen steigt, wodurch das zunehmende Potential der Gewebezellen, sich zu einem Karzinom zu entwickeln, angezeigt wird.

2. Verfahren gemäß Anspruch 1, wobei der Marker einer zellulären Wucherung ein Zellwucherungsantigen ist.

3. Verfahren gemäß Anspruch 2, wobei das Zellwucherungsantigen das Ki-67-Antigen ist.

4. Verfahren gemäß Anspruch 3, wobei das Ki-67-Antigen unter Verwendung eines Antikörpers, der dieses Antigen spezifisch erkennt, nachgewiesen wird.

5. Verfahren gemäß Anspruch 4, wobei der Antikörper MIB-1 ist.

6. Verfahren gemäß Anspruch 1, wobei der Marker einer programmierten Zelltodhemmung ein Antigen einer programmierten Zelltodhemmung ist.

7. Verfahren gemäß Anspruch 6, wobei das Antigen einer programmierten Zelltodhemmung ein BCL-2-Protein ist.

## Revendications

1. Procédé, conduit in vitro, d'évaluation de la potentialité des cellules du tissu épithélial cervical à évoluer vers un carcinome, comprenant :
la comparaison d'un premier pourcentage de cellules issu d'un prélèvement dudit tissu épithélial cervical qui co-expriment un premier marqueur de la prolifération cellulaire et un second marqueur de l'inhibition de la mort cellulaire programmée avec un pourcentage de cellules d'un modèle cellulaire qui co-exprime lesdits premier et second marqueurs,
avec pour effet que, avec une intensité croissante de pré-néoplasie, le nombre de cellules combinant les deux paramètres croît, ce fait indiquant la potentialitë d'évolution progressive desdites cellules tissulaires vers un carcinome.

2. Procédé selon la revendication 1, dans lequel ledit marqueur de la prolifération cellulaire est un antigène de la prolifération cellulaire.

3. Procédé selon la revendication 2, dans lequel ledit antigène de la prolifération cellulaire est l'antigène Ki-67.

4. Procédé selon la revendication 3, dans lequel l'antigène Ki-67 est détecté en utilisant un anticorps qui reconnaît spécifiquement ledit antigène.

5. Procédé selon la revendication 4, dans lequel ledit anticorps est MIB-1.

6. Procédé selon la revendication 1 dans lequel ledit marqueur de l'inhibition de la mort cellulaire programmée est un antigène de l'inhibition de la mort cellulaire programmée.

7. Procédé selon la revendication 6 dans lequel l'antigène de l'inhibition de la mort cellulaire programmée est la protéine BCL-2.
